# EUROPEAN PATENT SPECIFICATION

(11) **EP 2 249 812 B1**
(45) Date of publication and mention of the grant of the patent: **02.11.2022**
(21) Application number: 09707868.7
(22) Date of filing: 03.02.2009
(51) Int. Cl.: A61K 9/20, A61K 31/64

(54) **ORAL PHARMACEUTICAL FORMULATIONS CONTAINING GLICLAZIDE**
GLICLAZID ENTHALTENDE ORALE PHARMAZEUTISCHE FORMULIERUNGEN
FORMULATIONS PHARMACEUTIQUES ORALES CONTENANT DU GLICLAZIDE

(30) Priority: 05.02.2008 IT FI20080016
(43) Date of publication of application: 17.11.2010
(73) Proprietor: VALPHARMA S.p.A., 47899 Serravalle (SM)
(72) Inventor: VALDUCCI, Roberto, I-47039 Savignano Sul Rubicone (IT); ALIGHIERI, Tiziano, I-47900 Rimini (IT); AVANESSIAN, Serozh, I-47827 Villa Verucchio (IT)
(74) Representative: Valenza, Silvia
(86) International application number: PCT/EP2009/051182
(87) International publication number: WO 2009/098195

(56) References cited:
- EP-A- 1 741 435
- WO-A-2006/061697
- WO-A-2006/123213
- US-B1- 6 733 782
- DATABASE CA [Online] CHEMICAL ABSTRACTS SERVICE, COLUMBUS, OHIO, US; 20 October 2006 (2006-10-20), ZHANG, RONG ET AL: "Manufacture and dissolution test in vitro of gliclazide sustained -release pellet capsule" XP002528029 retrieved from STN Database accession no. 2006:1098308 & ZHONGGUO YAOYE , 15(10), 27-29 CODEN: ZYHAB5; ISSN: 1006-4931, 2006,
- DATABASE CA [Online] CHEMICAL ABSTRACTS SERVICE, COLUMBUS, OHIO, US; 4 April 2006 (2006-04-04), DING, LINHONG: "Enteric coated sustained release tablet of gliclazide and its preparation process" XP002528028 retrieved from STN Database accession no. 2006:308493 & CN 1 631 366 A (GUIZHOU SHENGJITANG PHARMACEUTICAL CO., LTD., PEOP. REP. CHINA) 29 June 2005 (2005-06-29)
- DATABASE CA [Online] CHEMICAL ABSTRACTS SERVICE, COLUMBUS, OHIO, US; 3 July 2007 (2007-07-03), LI, SHENGFEI: "Controlled-release preparation containing dimethylbiguanide HCl and gliclazide and its preparation method" XP002528027 retrieved from STN Database accession no. 2007:717220 & CN 1 985 819 A (BEIJING RUNDEKANG MEDICAL TECHNOLOGY CO., LTD., PEOP. REP. CHINA) 27 June 2007 (2007-06-27)

## Description

### Field of the invention

The present invention relates to pharmaceutical formulations for oral administration, and particularly to those containing gliclazide.

### State of the art

Pharmaceutical formulations for oral administration containing gliclazide as the active ingredient are well known.

The patent WO 0018373 describes the production of core tablets by mixing several types of hydroxypropyl methylcellulose (HPMC) and adding maltodextrin and other diluents.

The production method involves the following steps:
- mixing all the components except for the HPMC
- wet granulation with water
- drying and grading of the granules
- mixing first with the various types of HPMC to form the delayed-release matrix and then with lubricants
- compression.

A hydrophilic core tablet is thus obtained with a mechanism of release by means of gelification.

This type of formulation has considerable drawbacks, i.e.
- lengthy and costly process times;
- the need for ample production areas;
- the risk of segregating the active ingredient from the granules;
- contamination and toxicity of the production environments and for the operators involved, due to the dust forming in the various production stages and the considerable handling needed during the production process.

On the other hand, patents EP 1064935 and US 6319520 describe the formation of granules by means of thermoforming process using methacrylate derivatives, such as Eudragit RS, RL, E.

After granulation, which takes place at a temperature coming between 60 and 150°C, the resulting granules are compressed to produce core tablets.

The mechanism of release from these tablets is diffusive and it is pH-independent, in particular, in the case of Eudragit RS + Eudragit RL being used.

This type of formulation has the drawback of demanding a high-temperature extrusion process with a consequent risk of damaging the active ingredient and making the working conditions particularly hazardous.

Zhang et al. (STN Database Accession no. 2006:1098308; ZHONGGUO YAOYE, 15(10), 27-29) describes a granule composition comprising gliclazide, microcrystalline cellulose, ethylcellulose, stearic acid. The pellets are manufactured by extruding and spheronizing.

WO2006/061697A1 discloses once a da sustained release graaanules obtained by wet granulation comprising: a) gliclazide, b) the hydrophilic polymer HPMC K4M and c) HPMC K100VL. The granules are further blended with talc, colloidal silico dioxide and magnesium stearate before compression into tablets.

### Summary of the invention

It has now surprisingly been established that gliclazide can be mixed with hydrophilic ingredients in combination with a hydroxypropyl methylcellulose and lipophilic ingredients with a low melting point to obtain dust-free granules suitable, without further handling, for compression to form tablets.

Said tablets are characterised in that they release the active ingredients they contain over a period of 24 hours.

### Detailed description of the invention

Object of the present invention are tablets according to claim 1 and a process for preparing tablets according to claim 8. Oral formulations comprising gliclazide can be mixed with hydrophilic ingredients in combination with a hydroxypropyl methylcellulose and lipophilic ingredients with a low melting point.

The term hydrophilic ingredients is used to mean hydrophilic polymers (e.g. hydroxypropyl methylcellulose, hydroxyethylcellulose, hydroxypropyl cellulose, ethylene polyoxide, carbomer or mixtures thereof), mannitol, dibasic sodium phosphate, sorbitol, isomalt, or mixtures thereof.

According to the invention, HPMC is the preferred hydroxypropyl methylcellulose, in a solution at 2% w/w, with a viscosity corresponding to approximately 4000 cps. The term lipophilic elements with a low melting point is used herein to mean, for instance, stearic acid, carnauba wax, cetyl alcohol or cetyl stearyl alcohol. According to the invention, the above-mentioned components are contained in the following proportions (calculated weight to weight with respect to the total weight of the mixture):

| | |
|---|---|
| Gliclazide: | 10-30% |
| Stearic acid | 5-20% |
| Carnauba wax | 5-20% |

### HPMC

| | |
|---|---|
| (viscosity 2% w/w approx 4000 cps) | 5-25% |
| Mannitol | 10-50% |
| Dibasic sodium phosphate | 10-30% |
| Magnesium stearate | 0.1-5% |
| Anhydrous colloidal silica | 0.1-5% |
| HPMC 5 cps | 1 - 5% |
| PEG 4000 | 0.1 - 5% |
| Talc | 0.1-5% |
| Titanium dioxide | 0.1 - 5% |

### More preferably:

| | |
|---|---|
| Gliclazide: | 15-20% |
| Stearic acid | 10-20% |
| Carnauba wax | 5-15% |

### HPMC

| | |
|---|---|
| (viscosity 2% w/w approx 4000 cps) | 10-20% |
| Mannitol | 15-40% |
| Dibasic sodium phosphate | 10-20% |
| Magnesium stearate | 1-3% |
| Anhydrous colloidal silica | 1-3% |
| HPMC 5 cps | 1-4% |
| PEG 4000 | 0.1-3% |
| Talc | 0.5-3% |
| Titanium dioxide | 0.5-4% |

The above-described granules can be prepared in a single production step by granulation-extrusion at a temperature lower than 50°C, using standard granule production methods.

The granules are then mixed with the typical additives used in the preparation of tablets for oral administration, such as:

| | |
|---|---|
| lubricants | 0.5-2% |
| separating agents | 0.1-3% |
| anticaking agents | 0.1-10% |

and ultimately compressed.

If necessary, the tablets are subsequently coated with a soluble film (generally containing a low-viscosity hydroxypropyl methylcellulose or acrylic polymers, or colouring agents, etc) to facilitate their swallowing, make the tablets readily identifiable and facilitate subsequent procedures.

For the sake of completeness and a better understanding of the present invention, several examples of formulations according to the invention and their preparation are given below.

### Example 1

The components listed below are mixed together in an automatic drum mixer for 20 minutes at a turning speed of 4 rpm.

| | |
|---|---|
| Gliclazide | 3000 g (19.5%) |
| Mannitol | 4481 g (29.1%) |
| Stearic acid | 1539 g (10.0%) |
| Carnauba wax | 1539 g (10.0%) |
| Dibasic sodium phosphate | 2923 g (19.0%) |
| HPMC (viscosity 2% w/w approx 4000 cps) | 1904 g (12.4%) |

The resulting mixture is extruded using the Leistritz HP18 extruder in the following working conditions:

| | |
|---|---|
| temperature of the refrigerant: | 15°C |
| temperature of the extruder: | 50°C |
| temperature of the extruded products: | 53°C |

The granules thus obtained are mixed with the lubricants (magnesium stearate and anhydrous colloidal silica) and compressed into tablets containing doses of 30 and 60 mg of active ingredient.

| | | |
|---|---|---|
| Magnesium stearate: | 2 mg/tab (30 mg) | 4mg/cpr (60 mg) |
| Anhydrous colloidal silica: | 1 mg/tab (30 mg) | 2 mg/tab (60 mg) |

Characteristics of the tablets:

| | |
|---|---|
| mean weight of tablet: | 157 mg |
| shape of tablet: | oval |
| size of tablet: | 11x5.6 mm |

The tablets thus obtained are coated with a film in a GS 25 drum mixer using a titanium-dioxide-based white colouring agent in order to obtain a 10 mg increase in the weight of the tablet, under the following working conditions:

| | |
|---|---|
| air temperature at inlet: | 55°C |
| drum turning speed: | 12 rpm |
| nebulising pressure: | 1 bar |

The resulting tablets have a 24-hour release profile.

### Example 2

The components listed below are mixed in a suitable mixer:

| | | |
|---|---|---|
| Gliclazide | 733 g | (19,5%) |
| Mannitol | 1095 g | (29,1%) |
| Stearic acid | 376 g | (10.0%) |
| Carnauba wax | 376 g | (10.0%) |
| Dibasic sodium phosphate | 714 g | (19.0%) |
| HPMC (viscosity 2% w/w approx 4000 cps) | 465 g | (12.4%) |

The resulting mixture is extruded using the Leistritz HP18 extruder; the granules obtained are mixed with the lubricants and then compressed into tablets containing 30 and 60 mg of active ingredient, as previously described in example 1.

The tablets have a 24-hour release profile.

### Example 3

The components listed below are mixed in a suitable mixer.

| | | |
|---|---|---|
| Gliclazide | 733 g | (19.5%) |
| Isomalt | 950 g | (25.3%) |
| Stearic acid | 448 g | (11.9%) |
| Carnauba wax | 449 g | (11.9%) |
| Dibasic sodium phosphate | 700 g | (18.6%) |
| HPMC (viscosity 2% w/w approx 4000 cps) | 479 g | (12.8%) |

The mixture thus obtained is extruded using the Leistritz HP18 extruder; the resulting granules are mixed with the lubricants and subsequently compressed into tablets containing doses of 30 and 60 mg of active ingredient, as described previously in example 1.

The tablets have a 24-hour release profile.

As an example, the analytical results obtained on a batch of gliclazide 30 mg are given below.

| Time | Release |
|---|---|
| 1 h | 16% |
| 4h | 55% |
| 8h | 86% |
| 12h | 100% |

## Claims

1. Tablets comprising granules consisting of:
| | |
|---|---|
| Gliclazide: | 10-30% |
| Stearic acid | 5-20% |
| Carnauba wax | 5-20% |
**HPMC**
| | |
|---|---|
| (viscosity 2% w/w approx 4000 cps) | 5-25% |
| Mannitol | 10-50% |
| Dibasic sodium phosphate | 10-30% |
| Magnesium stearate | 0.1-5% |
| Anhydrous colloidal silica | 0.1-5% |
| HPMC 5 cps | 1 - 5% |
| PEG 4000 | 0.1 - 5% |
| Talc | 0.1 - 5% |
**wherein the granules are granulated extruded in a single production step at a temperature below 50°C, using standard granule production methods.**

2. Tablets according to claim 1, wherein the granules are consisting of:
| | |
|---|---|
| Gliclazide: | 15-20% |
| Stearic acid | 10-20% |
| Carnauba wax | 5-15% |
**HPMC**
| | |
|---|---|
| (viscosity 2% w/w approx 4000 cps) | 10-20% |
| Mannitol | 15-40% |
| Dibasic sodium phosphate | 10-20% |
| Magnesium stearate | 1-3% |
| Anhydrous colloidal silica | 1-3% |
| HPMC 5 cps | 1-4% |
| PEG 4000 | 0.1-3% |
| Talc | 0.5-3% |
| Titanium dioxide | 0.5-4% |

3. Tablets according to any of claims 1-2 wherein the granules are consisting of:
| | |
|---|---|
| Gliclazide | 19.5% |
| Mannitol | 29.1% |
| Stearic acid | 10.0% |
| Carnauba wax | 10.0% |
| Dibasic sodium phosphate | 19.0% |
| HPMC (viscosity 2% w/w approx 4000 cps) | 12.4%; or |
| Gliclazide | 19.5% |
| Isomalt | 25.3% |
| Stearic acid | 11.9% |
| Carnauba wax | 11.9% |
| Dibasic sodium phosphate | 18.6% |
| HPMC (viscosity 2% w/w approx 4000 cps) | 12.8% |

4. Tablets according to any of claims 1-3, comprising the granules in combination with additives chosen from among the following:
| | |
|---|---|
| lubricants | 0.5-2% |
| separating agents | 0.1-3% |
| anticaking agents | 0.1-10% |

5. Tablets according to claim 4, coated with a soluble film.

6. Tablets according to claim 4 comprising granules according to claim 3, in combination with magnesium stearate and anhydrous colloidal silica.

7. Tablets according to claim 6, coated with a soluble and/or colouring film.

8. A process for preparing tablets according to any of claims 1-7, said process comprising
preparing granules as defined in any of claims 1 -5 by a single production step of granulation-extrusion at a temperature below 50°C, using standard methods for the production of granules
the granules are then mixed with the typical additives used in the preparation of tablets for oral administration and ultimately compressed.

## Patentansprüche

1. Tabletten aufweisend Granulat bestehend aus:
| | |
|---|---|
| Gliclazid: | 10 - 30% |
| Stearinsäure | 5 - 20% |
| Carnaubawachs | 5 - 20% |
| HPMC (Viskosität 2 Gew.-% ca. 4000 cps) | 5 - 25% |
| Mannitol | 10 - 50% |
| Zweibasisches Natriumphosphat | 10 - 30% |
| Magnesiumstearat | 0,1 - 5% |
| Wasserfreie kolloidale Siliziumdioxid | 0,1 - 5% |
| HPMC 5 cps | 1 - 5% |
| PEG 4000 | 0.1 - 5% |
| Talkum | 0,1 - 5% |
wobei das Granulat in einem einzigen Produktionsschritt bei einer Temperatur von unter 50 °C unter Verwendung von Standardverfahren zur Herstellung von Granulat granuliert und extrudiert wird.

2. Tabletten nach Anspruch 1, wobei das Granulat aus folgenden Bestandteilen besteht:
| | |
|---|---|
| Gliclazid: | 15 - 20% |
| Stearinsäure | 10 - 20% |
| Carnaubawachs | 5 - 15% |
| HPMC (Viskosität 2 Gew.-% ca. 4000 cps) | 10 - 20% |
| Mannitol | 15 - 40% |
| Zweibasisches Natriumphosphat | 10 - 20% |
| Magnesiumstearat | 1 - 3% |
| Wasserfreie kolloidale Siliziumdioxid | 1 - 3% |
| HPMC 5 cps | 1 - 4% |
| PEG 4000 | 0.1 - 3% |
| Talkum | 0.5 - 3% |
| Titaniumdioxid | 0.5 - 4% |

3. Tabletten nach einem der Ansprüche 1 bis 2, wobei das Granulat aus folgenden Bestandteilen besteht
| | |
|---|---|
| Gliclazid | 19.5% |
| Mannitol | 29,1% |
| Stearinsäure | 10,0% |
| Carnaubawachs | 10,0% |
| Zweibasisches Natriumphosphat | 19,0% |
| HPMC (Viskosität 2 Gew.-% ca. 4000 cps) | 12,4 %; oder |
| Gliclazid | 19.5 % |
| Isomalt | 25.3 % |
| Stearinsäure | 11.9 % |
| Carnaubawachs | 11.9 % |
| Zweibasisches Natriumphosphat | 18.6 % |
| HPMC (Viskosität 2 Gew.-% ca. 4000 cps) | 12.8 % |

4. Tabletten nach einem der Ansprüche 1 bis 3, aufweisend das Granulat in Kombination mit Zusatzstoffen, die aus den folgenden ausgewählt sind:
| | |
|---|---|
| Gleitmittel | 0.5 - 2% |
| Trennmittel | 0,1 - 3% |
| Antibackmittel | 0,1 - 10% |

5. Tabletten nach Anspruch 4, überzogen mit einem löslichen Film.

6. Tabletten nach Anspruch 4, aufweisend Granulat nach Anspruch 3 in Kombination mit Magnesiumstearat und wasserfreiem kolloidalem Siliciumdioxid.

7. Tabletten nach Anspruch 6, beschichtet mit einem löslichen und/oder färbenden Film.

8. Verfahren zur Herstellung von Tabletten nach einem der Ansprüche 1 bis 7, wobei das Verfahren umfasst
Herstellung eines Granulats nach einem der Ansprüche 1 bis 5 durch einen einzigen Produktionsschritt der Granulationsextrusion bei einer Temperatur unter 50 °C unter Verwendung von Standardverfahren zur Herstellung von Granulaten das Granulat wird dann mit den typischen Zusatzstoffen für die Herstellung von Tabletten zur oralen Einnahme vermischt und schließlich gepresst.

## Revendications

1. Comprimés comprenant des granulés constitués de :
| | |
|---|---|
| Gliclazide : | 10 à 30 % |
| Acide stéarique | 5 à 20 % |
| Cire de carnauba | 5 à 20 % |
| HPMC (viscosité 2 % p/p environ 4000 cps) | 5 à 25 % |
| Mannitol | 10 à 50 % |
| Phosphate de sodium dibasique | 10 à 30 % |
| Stéarate de magnésium | 0,1 à 5 % |
| Silice colloïdale anhydre | 0,1 à 5 % |
| HPMC 5 cps | 1 à 5% |
| PEG 4000 | 0,1 à 5 % |
| Talc | 0,1 à 5 % |
dans lesquels les granulés sont extrudés en une seule étape de production à une température inférieure à 50 °C, en utilisant des méthodes standard de production de granulés.

2. Comprimés selon la revendication 1, dans lesquels les granulés sont constitués de :
| | |
|---|---|
| Gliclazide : | 15 à 20 % |
| Acide stéarique | 10 à 20 % |
| Cire de carnauba | 5 à 15 % |
| HPMC (viscosité 2 % p/p environ 4000 cps) | 10 à 20 % |
| Mannitol | 15 à 40 % |
| Phosphate de sodium dibasique | 10 à 20 % |
| Stéarate de magnésium | 1 à 3 % |
| Silice colloïdale anhydre | 1 à 3 % |
| HPMC 5 cps | 1 à 4 % |
| PEG 4000 | 0,1 à 3 % |
| Talc | 0,5 à 3 % |
| Dioxyde de titane | 0,5 à 4 % |

3. Comprimés selon l'une quelconque des revendications 1 à 2 dans lesquels les granulés sont constitués de :
| | |
|---|---|
| Gliclazide | 19,5 % |
| Mannitol | 29,1 % |
| Acide stéarique | 10,0 % |
| Cire de carnauba | 10,0 % |
| Phosphate de sodium dibasique | 19,0 % |
| HPMC (viscosité 2 % p/p environ 4000 cps) | 12,4 % ; ou |
| Gliclazide | 19,5 % |
| Isomalt | 25,3 % |
| Acide stéarique | 11,9 % |
| Cire de carnauba | 11,9 % |
| Phosphate de sodium dibasique | 18,6 % |
| HPMC (viscosité 2 % p/p environ 4000 cps) | 12,8 % |

4. Comprimés selon l'une quelconque des revendications 1 à 3, comprenant les granulés en combinaison avec des additifs choisis parmi les suivants :
| | |
|---|---|
| lubrifiants | 0,5 à 2 % |
| agents de séparation | 0,1 à 3 % |
| agents anti-mottant | 0,1 à 10 % |

5. Comprimés selon la revendication 4, enrobés d'un film soluble.

6. Comprimés selon la revendication 4 comprenant des granulés selon la revendication 3, en combinaison avec du stéarate de magnésium et de la silice colloïdale anhydre.

7. Comprimés selon la revendication 6, enrobés d'un film soluble et/ou colorant.

8. Procédé de préparation de comprimés selon l'une quelconque des revendications 1 à 7, ledit procédé comprenant
préparer des granulés tels que définis dans l'une quelconque des revendications 1 à 5 en une seule étape de production de granulation-extrusion à une température inférieure à 50 °C, en utilisant des méthodes standard pour la production de granulés
les granulés sont ensuite mélangés avec les additifs typiques utilisés dans la préparation de comprimés pour l'administration orale et enfin comprimés.
